(19) Europäisches Patentamt
European Patent Office
Office européen des brevets

(11) **EP 2 310 500 B1**

(12) **EUROPEAN PATENT SPECIFICATION**

(45) Date of publication and mention
of the grant of the patent:
**11.03.2015 Bulletin 2015/11**

(51) Int Cl.:
***C12N 15/87*** *(2006.01)* ***A61K 48/00*** *(2006.01)*

(21) Application number: **09798713.5**

(86) International application number:
**PCT/US2009/050726**

(22) Date of filing: **15.07.2009**

(87) International publication number:
**WO 2010/009252 (21.01.2010 Gazette 2010/03)**

(54) **METHODS FOR OPTIMIZING ELECTROPORATION**

VERFAHREN ZUR OPTIMIERUNG EINER ELEKTROPORATION

PROCÉDÉS D'OPTIMISATION DE L'ÉLECTROPORATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.07.2008 US 81924 P**

(43) Date of publication of application:
**20.04.2011 Bulletin 2011/16**

(73) Proprietor: **Maxcyte, Inc.
Gaithersburg, MD 20878 (US)**

(72) Inventor: **DZEKUNOV, Sergey
Gaithersburg
MD 20878 (US)**

(74) Representative: **Pearce, David Henry
Barker Brettell LLP
100 Hagley Road
Edgbaston
Birmingham B16 8QQ (GB)**

(56) References cited:
**US-A- 4 622 302        US-A1- 2003 199 050
US-A1- 2004 214 333    US-A1- 2008 076 144**

• **NEVILLE CRAWFORD AND NICOLAS CHRONOS: "Electro-encapsulating Drugs Within Blood Platelets: Local Delivery to Injured Arteries During Angioplasty", SEMINARS IN INTERVENTIONAL CARDIOLOGY, LONDON, GB, vol. 1, no. 1, March 1996 (1996-03), pages 91-102, XP008124798, ISSN: 1084-2764**

**Description**

**BACKGROUND OF THE INVENTION**

**I. FIELD OF THE INVENTION**

The present invention relates generally to methods for the introduction of chemical or biological agent into living cells or cell particles or lipid vesicles.

**II. DESCRIPTION OF RELATED ART**

The outcome of electroporation process - using an electrical field for loading living cells or cell particles or lipid vesicles with extracellular material - is largely controlled by two major parameters: the magnitude of applied electrical field (EF) pulse and the duration of the pulse. As long as the pulse magnitude is above a certain threshold level, an increase in either the magnitude or the duration of the pulse generally results in a greater accumulation of extracellular molecules inside a cell.

The threshold for applied electrical field is inversely proportional to the size of the cell, and is typically in the range 1-3 kV/cm for nucleated cells, 2-4 kV/cm for red blood cells, 5-7 kV/cm for platelets and 7 - 10 kV/cm for bacteria and yeast (Crawford and Chronos 1996).

Each electrical pulse applied to a cell suspension can be characterized by a certain amount of energy, which is equal to the product of voltage on the electrodes, current through the buffer, and duration of high voltage pulse. However, only a small percentage of applied electrical energy is spent on the useful work of modifying lipid membranes and moving extracellular materials into cells. This energy cannot be measured directly. The rest of electrical energy dissipates in the form of heat that is produced in the cell-surrounding media. Power dissipation that slightly heats the cell suspension is an inevitable consequence of applying EF, even though heating itself does not cause permeabilization of cells. The more conductive the buffer is, the more energy is wasted on heat production. All physiological media has relatively large amounts of chlorine, sodium and/or potassium. The presence of these ions determines the media conductivity, which is usually in the range 15-20 mS/cm.

U.S. Patent 5,676,646 discloses a flow electroporation apparatus with a flow cell comprising two electrodes separated by a non-conductive spacer, the spacer defining a flow path. The major problem with this flow cell as well as other prior art flow cells is that the surface area of the electrode is not sufficient to dissipate heat as the cells are being electroporated. Thus, the heat buildup in the prior art flow cells is very large as the cells are being electroporated. This heat build up can cause damage to cells and cell components and decrease the efficiency of the electroporation process.

Heating of the buffer puts a limitation on the amount of energy used for successful electroporation of a cell suspension because the corresponding temperature rise must not exceed 20-24 degrees above ambient, otherwise the cells and/or biological material may suffer permanent damage.

There is also another effect of the temperature increase in an EP sample. It is related to an increase in the electrical conductivity of the sample, which in simple salt solutions increases by about 2% per °C. Applied electrical field causes a current flow through the cell or particle suspension, which causes a temperature rise that translates into a conductivity increase and a greater current draw from the power source, and so on. If such positive feedback process is not interrupted (e.g., by switching the pulse off), the current increase proceeds in an avalanche-like manner and results in arcing and sample loss. This effect is mainly observed at relatively high field strengths (> 2 kV/cm).

The concept that a biologically active agent can be inserted into platelets with electroporation and that the agent will then exert an effect at a target site has been demonstrated and is documented in peer-reviewed literature. In these cited studies, platelets were treated with static systems that accommodated a maximum of 0.5 ml of cell suspension. U.S. Patent 7,186,559 discloses methods and apparatus that permits processing of tens or hundreds of milliliters of cell suspension with a rapid (minutes) throughput time, including platelets.

Electroporation of platelets requires even stronger electrical fields and therefore either the buffer conductivity or pulse width must be limited. On the other hand, it is well known from the literature (Authi *et al.,* 1989; Hughes and Crawford 1989; Crawford and Chronos 1996) that platelets are extremely sensitive to biochemical changes in their environment and should be kept under physiological conditions whenever possible. In addition, the physico-chemical changes in the environment associated with application of electrical field to a suspension of platelets may modulate the physiological state, activation properties, and biological function of platelets impacting the ability to deliver clinical effect. The buffers developed for platelet handling and electroporation having relatively high conductivity and very short electrical pulses are used (Pfliegler *et al.,* 1994).

In certain cases such as loading of nucleic acids by electroporation into primary or cultured cells, a long pulse (>100 us) is required (Wolf *et al.,* 1994; Rols and Teissie 1998) to transport large RNA or DNA molecules across cell membranes. Assuming that the observations of RNA loading by electroporation into nucleated cells can be carried over onto platelets, one has to use long electrical pulses to load platelets with RNA and any other large charged molecules. To achieve the best outcome of such process, a user would have to use very high voltages applied to highly conductive media for relatively long intervals of time. In practice, these three conditions are mutually exclusive and cannot be easily optimized all at once, mainly due to the risk of arcing. Therefore, there is a need for a simple and efficient method for electroporating chemical or biological agents without damaging the cells, liposomes, or vesicles beyond their ability to produce a clinical effect.

The document US2008076144 describes methods of measuring resistances of samples to be electroporated and utilizing the measured resistances in the electroporation.

## SUMMARY OF THE INVENTION

The present invention is as defined by the appended claims, and relates to a method for optimizing electroporation. The present invention is particularly suited for cells found in blood and other body tissues and fluids. The present invention also encompasses non-cellular derived lipid vesicles, liposomes and other lipid based drug delivery systems.

Embodiments of the invention include an electroporation method as defined by the appended claims and comprising (a) determining electroporation parameters such that during an electrical pulse a first time constant representative of electrical conductivity increase in electroporation medium ($t_1$) during the pulse is not less than a second time constant representative of capacitor discharge ($t_2$), wherein the pulse duration is less than either $t_1$ or $t_2$; and (b) applying one or more electrical pulses under the electroporation parameters to a sample to be electroporated. In certain aspects the electroporation parameters comprise buffer conductivity, power supply capacitance, electroporation chamber geometry, and electric field strength. In further aspects electroporation parameters can include one or more of the following: the buffer conductivity can be between 0, 0.1. 0.2, 0.3, 0.4, 0.5, 1, 1.5 to 1, 1.5, 2, 2.5, 3 Ohm/m including all values and ranges there between; the power supply capacitance can between 1, 10, 20, 30, 40, 50, 100, 200, 500 to 200, 300, 400, 500, 800, 1000, 5000, 10,000, 50,000, $10^4$, $10^5$, $10^6$ $\mu$F including all values and ranges there between; the electroporation chamber can have dimensions of length betwenn 0.1, 1, 10, 50 to 20, 40, 80, 100 cm including all values and ranges there between, width between 0.1, 0.5, 1, 5 to 1, 5, 10 cm including all values and ranges there between, and a gap between 0.001, 0.01, 0.1, 1, 5 to 0.1, 1, 5, 10 cm including all values and ranges there between; the electric field can be between 0.1, 1, 2.5, 5 to 2.5, 5, 10 kV/cm Including all values and ranges there between. In still further aspects, the electrical pulse is at least 0.5, 1, 5, 10, 15, 20, 25, 50, 100, 200, 500 $\mu$sec or longer and includes all values and ranges there between. The electrical pulse can be of a magnitude of 0.25, 0.5, 1, 2, 3, 4, 5, 6, 7, 8, 9, 10 to 5, 6, 7, 8, 9, 10, 11, 12, 13, 14, 15 kV/cm including all values and ranges there between. In yet another aspect the electrical pulse can be of a magnitude of 0.001, 0.01, 0.1, 1, 10, 100, 1000 to 10, 100, 1000, 10000 volts including all values and ranges there betweenIn one aspect the electrical pulse is of a magnitude of 5 kV/cm.

In certain aspects a sample comprises a living cell, a cell particle, or a lipid vesicle. In a further aspect, the cell is a blood cell or a platelet, or fragment or derivative thereof. The living cell, cell particle, or lipid vesicle can be loaded with a chemical or biological agent. The biologically active substance can be a nucleic acid or small molecule and the like.

An electroporated cell, cell particle, lipid vesicle, or other product may be produced using any of the described methods. In certain aspects, the electroporated cell, cell particle, or lipid vesicle is a platelet. A population of delivery vehicles including, but not limited to electroporated cells, cell particles, or lipid vesicles can have a loading efficiency of at least, at most, or about 50, 60, 70, 80, 90, 95, 99% including all values and ranges there between.

A further embodiment is directed to an electroporation method as defined by the appended claims and comprising (a) determining electroporation parameters such that during a decaying electrical pulse the rate of conductivity increase in an electroporation medium is lower that the rate of voltage decay; and (b) applying one or more electrical pulses under the electroporation parameters to a sample to be electroporated. In certain aspects, the rate of voltage decay is 1, 2, 3, 4, 5, 6, 7, 8, 9, 10 to 4, 5, 6, 7, 8, 9, 10, 11, 12 $\mu$s, ns, ms, or second (s) including all values and ranges there between.

Also disclosed herein is a method of treating a subject having or suspected of having a disease or condition comprising administering a product of any of the methods described in an amount that mitigates the disease or condition. In certain aspects the disease or condition is related to abnormal platelet function or levels. In certain aspects a product of the methods is a drug delivery vehicle and it is contemplated that a wide variety of known drugs can be delivered via loaded particles produced by the methods described. A disease or condition can include any disease or condition amenable to the delivery of a drug or agent via liposome particle, cell particle, or similar delivery vehicle that is prepared (e.g., loaded) by using electroporation methods. Examples of such diseases or conditions includes, but is not limited to thrombocytopenia, Gaucher's disease, aplastic anemia, alloimmune disorders, hemolytic-uremic syndrome, Bernard-Soulier syndrome, Glanzmann's thrombasthenia, Scott's syndrome, von Willebrand disease, Hermansky-Pudlak Syndrome, or hemophilia.

Also disclosed herein is a method of treating a subject having or suspected of having a disease or condition by administering an effective amount of a drug, a biologic or other bioactive molecule comprised in a particle produced by the methods described. In certain aspects the disease is an infectious disease, including but not limited to a bacterial, fungal, parasite, or virus infection. In a further aspect the bacterial infection is a mycobacterial infection. In still a further aspect the viral infection is a retroviral infection including but not limited to HIV infection. In another aspect the disease is an inflammatory disease or cancer or vascular occlusive disease.

Throughout this application, the term "cell" or "delivery vehicle" as it refers to a target of electroporation or a vehicle for delivery of a drug or therapeutic is meant to include human or animal cells in the biological sense. Platelets can be described as "cell-derived particles."

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only, since various changes and modifications within the scope of the invention as defined by the appended claims will become apparent to those skilled in the art from this detailed description.

## DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

FIG. 1 Results of a simulation of a current increase in the same chamber at different field strengths and short (left column) and long (right column) time scales.

FIG. 2 Reduction of capacitance to 100 $\mu$F allows application of a long pulse at 4 kV/cm.

FIG. 3 Reduction to 10 $\mu$F enables use of a shorter pulse at 8 kV/cm.

FIG. 4 Electrical field of 0.5 kV/cm was applied in two consecutive pulses 1 ms duration and 1 second apart to a conductive medium. There is a mild short-term conductivity increase in the beginning of each pulse.

FIG. 5 Electrical field of 1 kV/cm was applied in four consecutive pulses 1 ms duration and 1 second apart to a conductive medium. There is a noticeable conductivity increase during each pulse as well as with each subsequent pulse.

FIG. 6 Electrical field of 1.5 kV/cm was applied in four consecutive pulses 1 ms duration 1 second apart to a conductive medium. There is a noticeable conductivity increase during each pulse as well as with each subsequent pulse. The conductivity values at the beginning of pulses 2-4 are approximately the same as the values at the end of the pulses before them. This illustrates slow rates of buffer cooling during the intervals between the pulses.

FIG. 7 Electrical field of 2 kV/cm was applied in four consecutive pulses 1 ms duration 1 second apart to a conductive medium. At this field strength the sample conductivity rapidly increased during each pulse, exceeding 200 percent of its initial value. Arcing occurred during the pulses 3 and 4.

FIG. 8 Platelets were loaded with AllStars Negative Control siRNA (Qiagen, Inc.). The stock solution of siRNA was added to platelet suspension to final concentration of 1 $\mu$M, the sample was divided into "Coincubation" and "Electroporation" samples, and the latter was processed by application of electrical pulses on MaxCyte System. The power supply capacitance was set to 20 $\mu$F, field strength was 5 kV/cm.

**FIG. 9** Platelets were prepared as described for FIG. 8 and loaded with Alexa-488 (Invitrogen, Inc.) by electroporation.

## DETAILED DESCRIPTION OF THE INVENTION

The invention in its broadest sense is as defined in the independent claims.

Certain embodiments of the invention are directed to a technique for electroporation that allows for a delivery of long electrical pulses of high magnitude in highly conductive buffers and minimizes damage by electrical arc or a heat shock.

## I. ELECTROPORATION

The process of electroporation generally involves the formation of pores in a cell membrane, or in a vesicle, by the application of electric field pulses across a liquid cell suspension containing cells or vesicles. During the poration process, cells are often suspended in a liquid media and then subjected to an electric field pulse. The medium may be electrolyte, non-electrolyte, or a mixture of electrolytes and non-electrolytes. The strength of the electric field applied to the suspension and the length of the pulse (the time that the electric field is applied to a cell suspension) varies according to the cell type. To create a pore in a cell's outer membrane, the electric field must be applied for such a length of time and at such a voltage as to create a set potential across the cell membrane for a period of time long enough to create a pore.

Applying a voltage across a plasma membrane that exceeds a certain threshold level forms a pore in the membrane. If the strength of the applied electrical field and/or duration of exposure to it are properly chosen, the pores formed by the electrical pulse reseal after a short period of time, during which extracellular compounds have a chance to enter into the cell. However, excessive exposure of live cells to electrical fields can cause apoptosis and/or necrosis - processes that result in cell death.

Generally, the process of electroporation is often used for the transformation of bacteria, yeast, plant protoplasts, cultured cells and other cells or vesicles as a way of introducing some substance into a cell or a vesicle, such as loading it with a molecular probe, a drug that can change the cell's function, or pieces of DNA or forms of RNA, such as mRNA, siRNA or microRNA. This procedure is also highly efficient for the introduction of chemical or biological agents that specifically intervene in molecular pathways in tissue culture cells or primary cells, especially mammalian cells. For example, electroporation is used in the process of producing knockout mice, as well as in tumor treatment, gene therapy, and cell-based therapy.

During an electroporation process the electrical current flowing through a conductive media causes its heating and subsequent increase in its conductivity. If not properly controlled, such conductivity increase leads to drawing even more current from the power source and may lead to arching and loss of sample. This effect is typically observed at relatively high field strengths (> 2 kV/cm). However, electroporation of platelets, for example, requires relatively strong electrical fields and therefore either the buffer conductivity or pulse width can be limited. Many electroporation instruments deliver voltage pulses to a sample from capacitors precharged to a desired voltage. During a pulse, the voltage on a capacitor decreases, and this decrease can be viewed as compensation to the sample conductivity increase.

Many electroporation instruments deliver voltage pulses to a sample from capacitors, which are pre-charged to a desired voltage. A capacitor is an electrical/electronic device that can store energy in the electric field between a pair of conductors (called "plates"). The process of storing energy in the capacitor is known as "charging", and involves electric charges of equal magnitude, but opposite polarity, building up on each plate. A capacitor consists of two conductive electrodes, or plates, separated by a dielectric. Capacitors are used in electrical circuit and electronic circuits as energy-storage devices.

The capacitor's capacitance (C) is a measure of the amount of charge (Q) stored on each plate for a given potential difference or voltage (V) which appears between the plates: C = Q/V. In SI units, a capacitor has a capacitance of one farad when one coulomb of charge is stored due to one volt of applied potential difference between the plates. Since the farad is a very large unit, values of capacitors are usually expressed in microfarads ($\mu$F), nanofarads (nF), or picofarads (pF).

Conditions for compensating for capacitor voltage decrease relative to a sample conductivity increase are analyzed as follows.

The current through the buffer is represented by

$$\text{Equation 1: } I(t) = \frac{U(t)}{R(t)} = U(Tt)\sum(t)$$

where I(t) is current [A], U(t) is voltage [V], R(t) is resistance [Ohm] and E(t) is conductance [S].

The conductance change is represented by

$$\text{Equation 2:} \quad \sum(t) = \frac{A}{\lambda}\sigma(t)$$

where A is electrode surface (cm$^2$), $\lambda$ is distance between electrodes [cm], and $\sigma(t)$ is conductivity [mS/cm].

The conductivity change is represented by

$$\text{Equation 3:} \quad \sigma(t) = \sigma_0(1 + 0.02(\mathrm{T}(t) - \mathrm{T}_0))$$

where $\sigma_0$ is the conductivity at time zero ($T_0$) [S], and T(t) is the buffer temperature [K].

The temperature increase can be represented by

$$\text{Equation 4:} \quad \frac{dT(t)}{dt} = \frac{1}{\nu\kappa}\frac{dQ(t)}{dt}$$

where $\nu$ is buffer volume (cm$^3$), $\kappa$ is the heat capacity of the buffer [J/(cm$^3$K), and Q(t) is heat energy [J].

Heat generation can be represented by

$$\text{Equation 5:} \quad \frac{dQ(t)}{dt} = U(t)I(t)$$

Combining Eq. 1 through Eq. 3 results in

$$\text{Equation 6:} \quad I(t) = U(t)\frac{A}{\lambda}\sigma_0(1 + 0.02(T(t) - T_0))$$

Substituting Eq. 5 into Eq. 4 results in

$$\text{Equation 7:} \quad \frac{dT(t)}{dt} = \frac{1}{\nu\kappa}U(t)I(t)$$

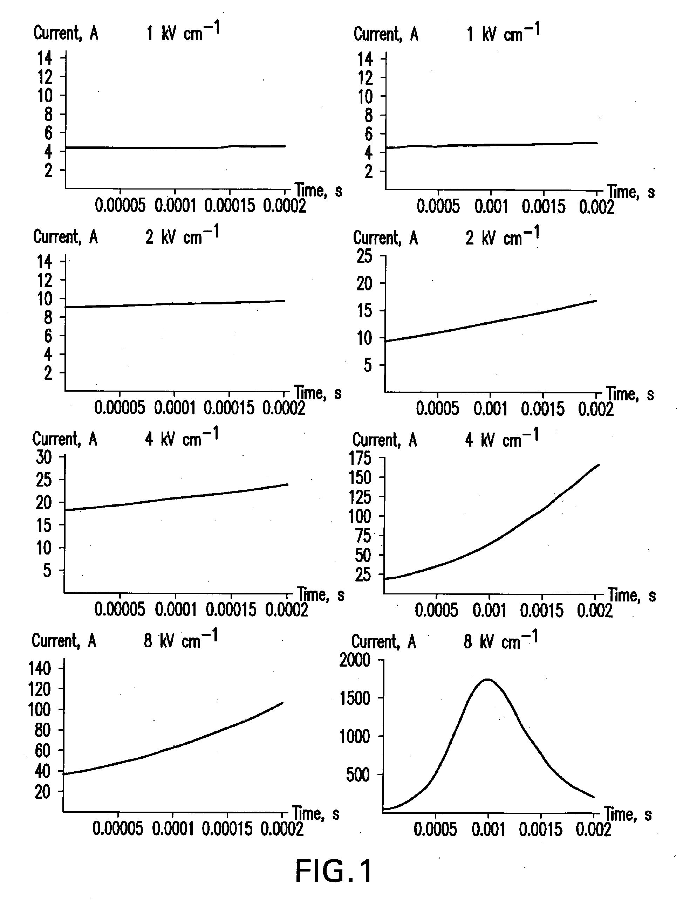

The system of Eq. 6 and Eq. 9 may not be solved analytically, only a numerical solution can be computed (FIG. 1). The equations also can be analyzed as follows.

Case 1: constant voltage. If the power supply has a very large capacitance or a DC battery, the voltage applied to sample is almost constant during a pulse:

Then differentiating Eq.6 over time results in:

Equation 8: $\dfrac{dI(t)}{dt} = \dfrac{A}{d}\sigma_0\left(0.02U(t)\dfrac{dT(t)}{dt} + \dfrac{dU(t)}{dt}(1+0.02(T(t)-T_0))\right)$

Substituting Eq.7 into Eq.8 results in:

Equation 9: $\dfrac{dI(t)}{dt} = \dfrac{A}{d}\sigma_0\left(0.02\dfrac{1}{v\kappa}U(t)^2 I(t) + \dfrac{dU(t)}{dt}(1+0.02(T(t)-T_0))\right)$

Under constant voltage conditions dU(t)/dt = 0, U(t) = $U_0$. Substituting these expressions into Equation 9 results in:

Equation 10: $\dfrac{dI(t)}{dt} = 0.02\dfrac{A}{\lambda v}\dfrac{\sigma_0}{\kappa}U_0^2 I(t) = 0.02\dfrac{\sigma_0}{\kappa}E^2 I(t)$

Solving the equation result in

Equation 11: $I(t) = I_0 e^{\left(\frac{t}{\tau 1}\right)}$

Equation 12: $I_0 = U_0\dfrac{A}{\lambda}\sigma_0$

Equation 13: $\dfrac{1}{\tau 1} = 0.02\dfrac{\sigma_0}{\kappa}E^2$

There is an exponential current increase due to a conductivity increase that is dependent mostly on the applied field strength.

Case 2: compensation. If the $\tau 1$, the time constant of conductivity increase, is matching $\tau 2$, the time constant of capacitor discharge, both processes are likely to compensate for each other during a short pulse (when duration of the pulse is less than either time constant). The discharge of a capacitor can be described by

Equation 14: $I(t) = -C\dfrac{dU(t)}{dt}$

where C is the electrical capacitance [F]

The time constant of this exponential process can be expressed in terms of initial conductivity and capacitance

$$\text{Equation 15:} \quad \frac{1}{\tau 2} = \frac{A\sigma_0}{\lambda C}$$

The compensation condition $\tau_1 = \tau_2$ becomes

$$\text{Equation 16:} \quad \frac{0.02}{\kappa} E^2 = \frac{A}{\lambda C}$$

The five variables in the Eq.16 can be expressed through other parameters (e.g. the ratio of chamber volume to the electrode gap $V/\lambda$ can be used instead of the electrode surface area A) without changing mechanism of the processes described above and ways to avoid arcing.

As an example, the buffers developed for platelet handling and electroporation having relatively high conductivity and very short electrical pulses are used; one probably has to use long electrical pulses to load platelets with RNA and any other large charged molecules. To achieve the best outcome of such process, a user would have to use very high voltages applied to highly conductive media for relatively long intervals of time. These three conditions are not easily met concurrently. A solution to this problem is the disclosed methods for electroporation that allow for a delivery of long electrical pulses of high magnitude in highly conductive buffers and minimizes the chance of damaging the cell or vesicle by electrical arc or a heat shock. In the present invention, during a pulse in electroporation, the voltage on a capacitor decreases, and this decrease can be viewed as compensation to the sample conductivity increase.

Typically five variables can be aligned to achieve either constant or decreasing current in a processing chamber. These variables together define how fast the current through the buffer will decrease due to the capacitor discharge and/or increase due to a change in the buffer conductivity. Typically, the dimensions of the processing chamber (A and $\lambda$) are predefined and the field strength (E) is dictated by the electroporation protocol. This leaves conductivity and capacitance as the most convenient variables to be manipulated with capacitance being the most flexible variable due to the existence of upper limits on conductivity as a result of maximal current rating on hardware. Typically, variables are optimized to achieve constant current in a processing chamber. In the present invention, any of the variables could be adjusted to achieve the desired compensation effect.

The data for FIG.1 were generated using Mathematica™ software (Wolfram Research, CA) as a numerical solution of a system of two differential equations.

**Code example.** Specify heat capacity of water k [J/(K mL), cuvette width w [cm], cuvette height h [cm], cuvette gap g [cm], capacitance c [F], pulse voltage u0 [V], initial buffer conductivity S0 [S/cm], pulse width tmax [s], y is the temperature

```
k = 4.18; w = 0.5; h = 0.5; g = 0.2;
c = 0.00001; u0 = 1600; S0 = 0.018; tmax = 0.0002;
solution = NDSolve[
    {u'[t] == - u[t]/c  wh/g  S0 (1 + 0.02 (y[t] - 25)),
     y'[t] == - c/(k w h g) u[t] u'[t], u[0] == u0, y[0] == 25},
    {u, y}, {t, 0, tmax}];
Plot[Evaluate[-c ∂_t u[t] /. solution], {t, 0, tmax},
    PlotRange → {0, 150}, AxesLabel → {Time, s, Current, A}, PlotLabel → 8 kV cm^{-1}];
```

[K]:

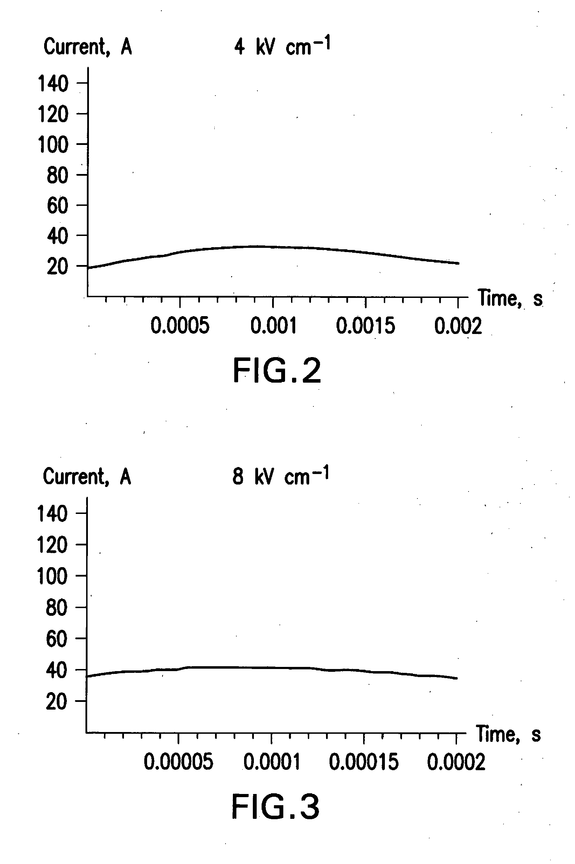

FIG.1 shows results of a simulation of a current increase in the same chamber at different field strengths and short (left column) and long (right column) time scales. Capacitance is reduced to 100 $\mu$F and allows application of a long pulse at 4 kV/cm (FIG. 2) and a reduction to 10 $\mu$F enables use of a shorter pulse at 8 kV/cm (FIG. 3).

## II. ELECTROPORATION TARGETS

Targets for electroporation include a number of cell types or particles derived from a number of organisms and sources. In some embodiments, the target can be nucleated or anucleated cells or particles. Cells or particles can be primary cells or a cell line or a particle derived therefrom. For example, a target may be prokaryotic, yeast, insect, mammalian, rodent, hamster, primate, human, bird, plant cells, or portions/fragments thereof. In certain aspects, the present invention relates to methods for the introduction of chemical or biological agents into various types of living cells or cell particles or synthetic vesicles or liposomes. More particularly, the present invention relates to a method for the introduction of chemical or biological agents into cells or cell particles. These electroporation targets can be utilized as a drug delivery system (an "intelligent liposome") to target a site of infection, metastasis, or other pathologic lesion - particularly if drug is otherwise toxic if not in a delivery vehicle.

If the patient population is infected with a virus and may exhibit viremia, the electroporation target can be allogeneic rather than autologous or synthetic in order to not exacerbate the patient's condition. Allogeneic cells or material can be obtained from standard sources (hospital services). Donors can be screened using histories and blood tests for infectious diseases.

### A. Electroporation Target Manufacture/Collection

Compositions described herein can be used in therapeutic applications. They are either isolated from collected samples to make a therapeutic dose. One example of the therapeutic use of the compositions described herein is formulating a therapeutic agent in appropriate buffer at a required concentration and processing the formulation using systems such as the MaxCyte system. If the formulated drug and electroporation target are sterile filled into a container with appropriate port(s), this can be run through a closed, sterile system in routine laboratory environment (filling room-like controlled environment not needed). The process can be completed within 2 to 3 hours. Performance variables of the system are generated in real time and will assist QC operations.

Typically, manufacturing of the drug-loaded target can be done at a central facility or at the point(s)-of care. If there is to be a central facility (or several regional ones), the stability of the drug-loaded target is an important factor. Stability for at least several days could support a custom order operation. In a point-of-care system, formulated drug would be supplied to the sites where therapy is required. Targets or delivery vehicles can be obtained at those sites and the final manufacturing step carried out processing facility by technical personnel using detailed standard operating procedures (SOPs). This would be analogous to final preparation of transfusion products on site. In this case, the final product stability is not critical.

### B. Therapeutic Applications

The disclosure further encompasses methods for delivering therapeutic agents using an electroporated entity or target, (e.g., cell, liposome, platelet, or derivative thereof) as a delivery vehicle. The active agent formulations produced using the methods described herein typically have a sustained effect and lower toxicity allowing less frequent administration and an enhanced therapeutic index. Therapeutic agents are delivered by first preparing platelets, cells, liposomes, or segments thereof loaded with at least one therapeutic agent, as obtained according to the methods described herein.

In certain embodiments of the present invention, substances or drugs can be introduced into a delivery vehicle (i.e., an electroporation target). The delivery vehicles can also be loaded with bioactive compounds. Examples of suitable bioactive compounds include, but are not limited to, stabilizing agents, tracers, fluorescent tags and other imaging substances such as radiolabels, cryoprotectants, nucleic acids, and bioactive materials. Bioactive materials particularly suited to incorporation into platelets include, but are not limited to, hemostatic effectors, wound healing factors, anti-cancer agents, cytokines and drugs. Suitable bioactive materials also include therapeutic and prophylactic agents. Examples of bioactive materials include, but are not limited to, proteins and peptides (synthetic, natural, and mimetics), oligonucleotides (anti-sense, ribozymes, etc.), nucleic acids (e.g., inhibitory RNA, siRNA, miRNA, expression vectors, etc.), viral vectors, hemotherapeutic agents, anti-cancer drugs, antiinflammatory drugs, anti-fungal drugs, anti-viral drugs, anti-microbial drugs, thrombomodulating agents, immunomodulating agents and the like.

Thrombus dissolving substances can be introduced by delivery vehicles, such as tissue plasminogen activator, urokinase or streptokinase and the like can be introduced into a population of delivery vehicles. Tissue plasminogen activator is sold under the trademark ACTIVASE® by Genentech Corporation, South San Francisco, Calif. These delivery vehicles loaded with the thrombus dissolving substances can then be introduced into a patient who is suffering from a thrombus blocking a blood vessel. Because the delivery vehicles contain active thrombus dissolving enzymes, the thrombus is then dissolved. The thrombus dissolving substances can be introduced into the delivery vehicles by a variety of methods with the most preferable method according to the present invention.

It is to be understood that other drugs can be introduced into the delivery vehicle or other cells for delivery to damaged tissue. These drugs include, but are not limited to, smooth muscle inhibitors, antiinfective agents (*e.g.,* antibiotics, antifungal agents, antibacterial agents, antiviral agents), chemotherapeutic/antineoplastic agents and the like.

In one embodiment, the drug is an antiinfective. Antiinfectives are agents that act against infections, such as bacterial, mycobacterial, fungal, viral or protozoal infections. Antiinfectives covered by the invention include but are not limited to aminoglycosides (e.g., streptomycin, gentamicin, tobramycin, amikacin, netilmicin, kanamycin, and the like), tetracyclines (e.g., chlortetracycline, oxytetracycline, methacycline, doxycycline, minocycline and the like), sulfonamides (e.g., sulfanilamide, sulfadiazine, sulfamethaoxazole, sulfisoxazole, sulfacetamide, and the like), paraaminobenzoic acid, diaminopyrimidines (e.g., trimethoprim, often used in conjunction with sulfamethoxazole, pyrazinamide, and the like), quinolones (e.g., nalidixic acid, cinoxacin, ciprofloxacin and norfloxacin and the like), penicillins (e.g., penicillin G, penicillin V, ampicillin, amoxicillin, bacampicillin, carbenicillin, carbenicillin indanyl, ticarcillin, azlocillin, mezlocillin, piperacillin, and the like), penicillinase resistant penicillin (e.g., methicillin, oxacillin, cloxacillin, dicloxacillin, nafcillin and the like), first generation cephalosporins (e.g., cefadroxil, cephalexin, cephradine, cephalothin, cephapirin, cefazolin, and the like), second generation cephalosporins (e.g., cefaclor, cefamandole, cefonicid, cefoxitin, cefotetan, cefuroxime, cefuroxime axetil; cefmetazole, cefprozil, loracarbef, ceforanide, and the like), third generation cephalosporins (e.g., cefepime, cefoperazone, cefotaxime, ceftizoxime, ceftriaxone, ceftazidime, cefixime, cefpodoxime, ceftibuten, and the like), other beta-lactams (e.g., imipenem, meropenem, aztreonam, clavulanic acid, sulbactam, tazobactam, and the like), betalactamase inhibitors (e.g., clavulanic acid), chlorampheriicol, macrolides (e.g., erythromycin, azithromycin, clarithromycin, and the like), lincomycin, clindamycin, spectinomycin, polymyxin B, polymixins (e.g., polymyxin A, B, C, D, E1 (colistin A), or E2, colistin B or C, and the like) colistin, vancomycin, bacitracin, isoniazid, rifampin, ethambutol, ethionamide, aminosalicyclic acid, cycloserine, capreomycin, sulfones (e.g., dapsone, sulfoxone sodium, and the like), clofazimine, thalidomide, or any other antibacterial agent that can be lipid encapsulated.

In certain aspects, anti-microbials include anti-mycobacterials, including, but not limited to isoniazid, rifampin, streptomycin, rifabutin, ethambutol, pyrazinamide, ethionamide, aminosalicylic and cycloserine.

Antiinfectives can include antifungal agents, including polyene antifungals *(e.g.,* amphotericin B, nystatin, natamycin, and the like), flucytosine, imidazoles *(e.g.,* n-ticonazole, clotrimazole, econazole, ketoconazole, and the like), triazoles (e.g., itraconazole, fluconazole, and the like), griseofulvin, terconazole, butoconazole ciclopirax, ciclopirox olamine, haloprogin, tolnaftate, naftifine, terbinafine, or any other antifungal that can be lipid encapsulated or complexed. Discussion and the examples are directed primarily toward amikacin but the scope of the application is not intended to be limited to this antiinfective. Combinations of drugs can be used.

In certain aspects, antiinfecives include anti-virals including but not limited to anti-herpes agents such as acyclovir, famciclovir, foscamet, ganciclovir, acyclovir, idoxuridine, sorivudine, trifluridine, valacyclovir and vidarabine; antiretroviral agents such as ritonavir, didanosine, stavudine, zalcitabine, tenovovir and zidovudine; and other antiviral agents such as, but not limited to, amantadine, interferon-alpha, ribavirin and rimantadine.

Also included as suitable antiinfectives used in the drug formulations of the present disclosure are pharmaceutically acceptable addition salts and complexes of drugs. In cases wherein the compounds may have one or more chiral centers, unless specified, the present disclosure comprises each unique racemic compound, as well as each unique nonracemic compound.

In aspect aspect, an active agent is an antineoplastic drug. Currently, there are approximately twenty recognized classes of approved antineoplastic drugs. The classifications are generalizations based on either a common structure shared by particular drugs, or are based on a common mechanism of action by the drugs. A partial listing of some of the commonly known commercially approved (or in active development) antineoplastic agents by classification is as follows:

Structure-Based Classes include Fluoropyrimidines--5-FU, Fluorodeoxyuridine, Ftorafur, 5'-deoxyfluorouridine, UFT, S-1 Capecitabine; Pyrimidine Nucleosides--Deoxycytidine, Cytosine Arabinoside, 5-Azacytosine, Gemcitabine, 5-Azacytosine-Arabinoside; Purines--6-Mercaptopurine, Thioguanine, Azathioprine, Allopurinol, Cladribine, Fludarabine, Pentostatin, 2-Chloro Adenosine; Platinum Analogues--Cisplatin, Carboplatin, Oxaliplatin, Tetraplatin, Platinum-DACH, Ormaplatin, CI-973, JM-216; Anthracyclines/Anthracenediones-Doxorubicin, Daunorubicin, Epirubicin, Idarubicin, Mitoxantrone; Epipodophyllotoxins--Etoposide, Teniposide; Camptothecins--Irinotecan, Topotecan, 9-Amino Camptothecin, 10,11-Methylenedioxy Camptothecin, 9-Nitro Camptothecin, TAS 103, 7-(4-methyl-piperazino-methylene)-10,11-ethylenedioxy-20(S)-camptothecin, 7-(2-N-isopropylamino)ethyl)-20(S)-camptothecin; Hormones and Hormonal Analogues--Diethylstilbestrol, Tamoxifen, Toremefine, Tolmudex, Thymitaq, Flutamide, Bicalutamide, Finasteride, Estradiol, Trioxifene, Droloxifene, Medroxyprogesterone Acetate, Megesterol Acetate, Aminoglutethimide, Testolactone and others; Enzymes, Proteins and Antibodies--Asparaginase, Interleukins, Interferons, Leuprolide, Pegaspargase, and others; Vinca Alkaloids--Vincristine, Vinblastine, Vinorelbine, Vindesine; Taxanes--Paclitaxel, Docetaxel.

Mechanism-Based Classes incude Antihormonals-- Anastrozole; Antifolates--Methotrexate, Aminopterin, Trimetrexate, Trimethoprim, Pyritrexim, Pyrimethamine, Edatrexate, MDAM; Antimicrotubule Agents--Taxanes and Vinca Alkaloids; Alkylating Agents (Classical and Non-Classical)-Nitrogen Mustards (Mechlorethamine, Chlorambucil, Melphalan, Uracil Mustard), Oxazaphosphorines (Ifosfamide, Cyclophosphamide, Perfosfamide, Trophosphamide), Alkylsulfonates (Busulfan), Nitrosoureas (Carmustine, Lomustine, Streptozocin), Thiotepa, Dacarbazine and others; Antimetabolites--Purines, pyrimidines and nucleosides, listed above; Antibiotics--Anthracyclines/Anthracenediones, Bleomycin, Dactinomycin, Mitomycin, Plicamycin, Pentostatin, Streptozocin; Topoisomerase Inhibitors-Camptothecins (Topo I), Epipodophyllotoxins, m-AMSA, Ellipticines (Topo II); Antivirals--AZT, Zalcitabine, Gemcitabine, Didanosine, and others; Miscellaneous Cytotoxic Agents-siRNA, miRNA, Hydroxyurea, Mitotane, Fusion Toxins, PZA, Bryostatin, Retinoids, Butyric Acid and derivatives, Pentosan, Fumagillin, and others.

Antiangiogenic drugs can be incorporated into the platelets. Antiangiogenic drugs include, but are not limited to, AGM-1470 (TNP-470) or antagonists to one of its receptors, MetAP-2; growth factor antagonists, or antibodies to growth factors (including VEGF or bFGF); growth factor receptor antagonists or antibodies to growth factor receptors; inhibitors of metalloproteinases including TIMP, batimastat (BB-94), and marimastat; tyrosine kinase inhibitors including genistein and SU5416; integrin antagonists including antagonists alphaVbeta3/5 or antibodies to integrins; retinoids including retinoic acid or the synthetic retinoid fenretinide; steroids $11\alpha$-epihydrocortisol, corteloxone, tetrahydrocortisone and $17\alpha$-hydroxyprogesterone; protein kinase inhibitors including staurosporine and MDL 27032; vitamin D derivatives including 22-oxa-1 alpha, and 25-dihydroxyvitamin D3; arachidonic acid inhibitors including indomethacin and sulindac; tetracycline derivatives including minocycline; thalidomide and thalidomide analogs and derivatives; 2-methoxyestradiol; tumor necrosis factor-alpha; interferon-gamma-inducible protein 10 (IP-10); interleukin 1 and interleukin 12; interferon alpha, beta or gamma; angiostatin protein or plasminogen fragments; endostatin protein or collagen 18 fragments; proliferin-related protein; group B streptococcus toxin; CM101; CAI; troponin I; squalamine; nitric oxide synthase inhibitors including L-NAME; thrombospondin; wortmannin; amiloride; spironolactone; ursodeoxycholic acid; bufalin; suramin; tecogalan sodium; linoleic acid; captopril; irsogladine; FR-118487; triterpene acids; castanospermine; leukemia inhibitory factor; lavendustin A; platelet factor-4; herbimycin A; diaminoantraquinone; taxol; aurintricarboxylic acid; DS-4152; pentosan polysulphite; radicicol; fragments of human prolactin; erbstatin; eponemycin; shark cartilage; protamine; Louisianin A, C and D; PAF antagonist WEB 2086; auranofin; ascorbic ethers; and sulfated polysaccharide D 4152.

Genes to be targeted using nucleic acid agents using the methods of the invention include, without limitation, those whose expression is correlated with an undesired phenotypic trait. Thus, genes relating to cancer, rheumatoid arthritis and viruses can be targeted. Cancer-related genes include oncogenes (e.g., K-ras, c-myc, bcr/abl, c-myb, c-fms, c-fos and cerb-B), growth factor genes (e.g., genes encoding epidermal growth factor and its receptor, fibroblast growth factor-binding protein), matrix metalloproteinase genes (e.g., the gene encoding MMP-9), adhesion-molecule genes *(e.g.,* the gene encoding VLA-6 integrin), tumor suppressor genes (e.g., bcl-2 and bcl-X1), angiogenesis genes, and metastatic genes. Rheumatoid arthritis-related genes include, for example, genes encoding stromelysin and tumor necrosis factor. Viral genes include human papilloma virus genes (related, for example, to cervical cancer), hepatitis B and C genes, and cytomegalovirus (CMV) genes (related, for example, to retinitis). Numerous other genes relating to these diseases or others might also be targeted. In certain embodiments nucleic acids can target mRNA encoding c-myc, VEGF, CD4, CCR5, gag, MDM2, Apex, Ku70, or ErbB2.

Methods of gene regulation include administering siRNA, miRNA, and other inhibitory nucleic acids; administration of nucleic acid encoding a therepaeutic nucleic acid, protein, or peptide. In another aspect, the disclosure provides a method of preparing and/or administering to the subject a dosage of a therapeutic inhibitory oligonucleotide or nucleic acid (antisense, ribozyme, siRNA, dsRNA) molecule wherein the administered nucleic acid inhibits a biological process such as transcription or translation. The present disclosure provides methods of administering one or more therapeutic nucleic acid molecules to a subject, using a nucleic acid delivery vehicle prepareed using the methods described, to bring about a therapeutic benefit to a subject. As used herein, a "therapeutic nucleic acid molecule" or "therapeutic nucleic acid" is any nucleic acid (e.g., DNA, RNA, non-naturally occurring nucleic acids and their analogues such as peptide nucleic acids, and their chemical conjugates) that, as a nucleic acid or as an expressed nucleic acid or polypeptide, confers a therapeutic benefit to a subject. The subject preferably is mammalian such as a mouse, and more preferably is a human being.

The agents, including thrombus dissolving substances, can be introduced into the delivery vehicle by a variety of methods with the most preferable method being according to the methods of the present invention.

The dosage of any compositions will vary depending on the symptoms, age and body weight of the patient, the nature and severity of the disorder to be treated or prevented, the route of administration, and the form of the subject composition. Any of the subject formulations may be administered in a single dose or in divided doses. Dosages for the compositions may be readily determined by techniques known to those of skill in the art or as taught herein.

In certain embodiments, the dosage of the subject compounds will generally be in the range of about 0.001, 0.01, 1, 5, 10 pg/ng/mg to about 0.1, 1, 5, 10 pg/ng/mg/g per kg body weight, including all values and ranges there between.

The present disclosure also includes a method of treating a patient in need of a therapeutic agent comprising administering to the patient an effective amount of platelets containing the therapeutic agent.

A number of disease states or conditions are related to abnormal platelet function or levels. These diseases or conditions include, but are not limited to thrombocytopenia *(e.g.,* Idiopathic thrombocytopenic purpura, thrombotic thrombocytopenic purpura, drug-induced thrombocytopenia, heparin-induced thrombocytopenia (HIT)), Gaucher's disease, aplastic anemia, fetomaternal alloimmune thrombocytopenia, HELLP syndrome, hemolytic-uremic syndrome, chemotherapy, Dengue, alpha-delta platelet storage pool deficiency (αδSPD), thrombocytosis (e.g., benign essential thrombocytosis), Bernard-Soulier syndrome, Glanzmann's thrombasthenia, Scott's syndrome, von Willebrand disease, Hermansky-Pudlak Syndrome, decreased cyclooxygenase activity (induced or congenital), storage pool defects (acquired or congenital), haemophilia, atherosclerosis, coronary artery disease, myocardial infarction, cerebrovascular disease, Stroke, CVA (cerebrovascular accident), peripheral artery occlusive disease (PAOD), angiogenic disorders, or cancer (e.g., delivery of anti-angiogenic substances).

## 111. DESCRIPTION OF A REPRESENTATIVE ELECTROPORATION DEVICE

Electroporation is done with electroporators, appliances that create the electric current and send it through the cell solution. The solution is pipetted into a glass or plastic cuvette that usually has two aluminum electrodes on its sides. For instance, for bacterial electroporation, a suspension of around 50 microliters is usually used. Prior to electroporation it is mixed with the plasmid to be transformed. The mixture is pipetted into the cuvette, the voltage is set on the electroporator (several hundred volts is often used) and the cuvette is inserted into the electroporator. Immediately after electroporation 1 milliliter of liquid medium is added to the bacteria (in the cuvette or in an eppendorf tube), and the tube is incubated at the bacteria's optimal temperature for an hour or more and then it is spread on an agar plate.

The flow electroporation devices that can be used in conjunction with the present invention is, in one embodiment, comprised of the following: instrument (electronics module (power box and tower)); computer (communicates with electronics module and actually runs the process in real time and manages process-associated data); monitor (displays graphical user interface [GUI] and enables user interaction); and disposable set or processing chamber (component in which flow electroporation of cell suspension occurs).

In certain embodiments, the present invention uses flow electroporation to overcome the practical limitations in respect to the number of cells that can be electroporated, the time in which they can be electroporated and the volume of solution in which they are suspended that attend to static or batch electroporation methods. With this method, a cell suspension is passed across parallel bar electrodes that are contained in a flow cell that is preferably disposable. It is to be understood that different configurations of flow cells can be used in the present invention. During this passage, the cells are subjected to electrical pulses with predetermined characteristics. The molecule of interest then diffuses into the cell following concentration and/or electrical gradients. The present invention is optionally capable of subjecting the cells to a range of electric field strengths. Generally speaking, field strengths useful in the present invention are greater than approximately 5 kV/cm; preferably, approximately 5 kV/cm to 7 kV/cm.

The process is initiated by attaching the flow cell with solutions and cell suspensions in the containers with the necessary fluids and samples. The flow cell snaps into place and is secured by closing a hinged panel. Priming solution (saline) and cell suspension are introduced by providing the required commands to the electroporation system, which controls operation of the pump and pinch valves. As the cells transit the flow path between electrodes, electric pulses of the chosen voltage, duration, and frequency are applied. Product and waste fluids are collected in ' the designated containers.

The user inputs the desired voltage and other parameters into the flow electroporation system. As noted above, a range of settings is optionally available. The computer communicates to the electronics in the tower to charge the capacitor bank to the desired voltage. Appropriate switches then manipulate the voltage before it is delivered to the flow path to create the electric field (the switches provide alternating pulses or bursts to minimize electrode wear brought on by prolonged exposure to the electric field). The voltage is delivered according to the duration and frequency parameters set into the flow electroporation system by the operator or as determined in accordance with the inventive method. Details of an example of a flow electroporation system is described in U.S. Patent 7,186,559.

Electroporation parameters will be determined using the procedures and methods described herein. Aspects of the invention can be used in context with static and flow electroporation systems. The dimension of the electroporation chambers will be considered in determining the parameter for optimal electroporation.

The electroporation arts are replete with ex vivo means of transfecting biological cells and vesicles. For example, U.S. Pat. No. 5,720,921 to Meserol discloses an electroporation chamber that is designed as a continuous flow chamber wherein vesicles are transferred to the chamber, electroporated and flushed out after electroporation pulses are applied. Other flow chambers include U.S. Patent 5,612,207 and 6,623,964, and U.S. Patent publication 2001/0001064.

Other electroporation chambers have also been disclosed wherein continuous flow of the medium carrying the vesicles is not used but the electroporation chamber device includes various elements. For example, U.S. Patent. 4,906,576 discloses a chamber having among other elements a magnetic core. U.S. Patent 6,897,069 discloses an electroporation sample chamber with removable electrodes. Other chambers are cuvette style for handling small samples. Still other chambers such as disclosed in WO04/083,379 provide for larger volumes.

Electrodes of electroporation chambers can comprise two or more "plate" electrodes. The electrode plates can comprise any useful biocompatible and conductive material including titanium and gold. The plates can comprise a width dimension that is generally greater than the distance, or gap, between opposing electrodes, and even more preferably greater than twice the gap distance. The electrode plate can be addressable with an electric pulse as determined by the present invention. The electrodes can comprise an array of between 1 and 100 cathodes and 1 and 100 anodes, there always being an even number of cathodes and anodes so as to form pairs of positive and negative electrodes.

The cathode and anode electrodes can be space on opposing interior sides of the reservoir at a distance of between 0.4 and 1 cm apart.

Each pair of said anodes and cathodes can be energized at a load resistance (in Ohms) depending upon the chamber size.

## IV. EXAMPLES

The following examples are included to further illustrate various aspects of the invention. It should be appreciated by those of skill in the art that the techniques disclosed in the examples that follow represent techniques and/or compositions discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention, which is as defined by the appended claims.

### EXAMPLE 1

### CONDUCTIVITY CHANGES ASSOCIATED WITH HIGH VOLTAGE PULSES

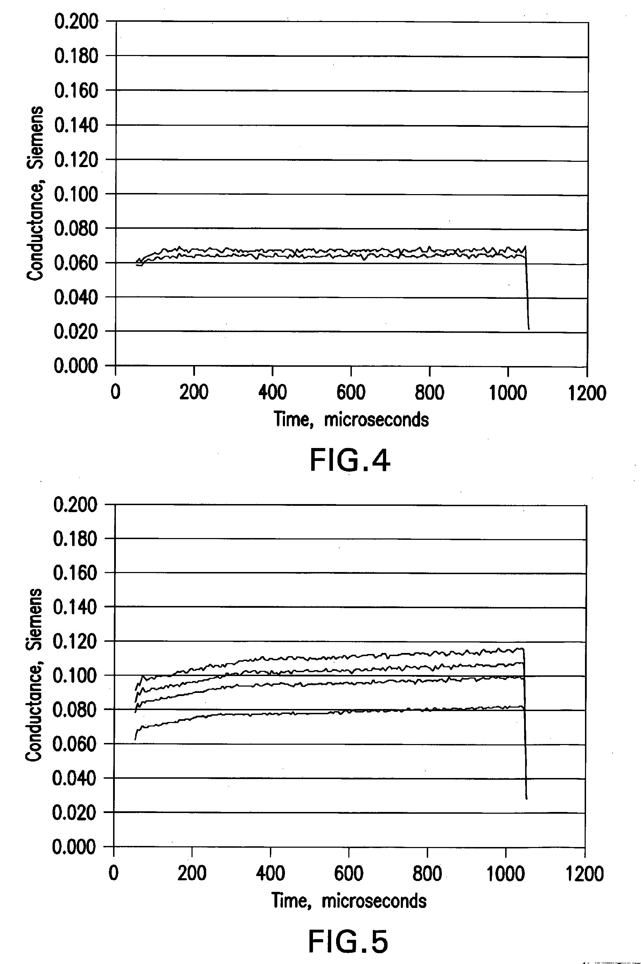

An electrical field of 0.5 kV/cm was applied in two consecutive pulses 1 ms duration and 1 second apart to a conductive medium (FIG. 4). There is a mild short-term conductivity increase in the beginning of each pulse.

An electrical field of 1 kV/cm was applied in four consecutive pulses 1 ms duration and 1 second apart to a conductive medium (FIG. 5). There is a noticeable conductivity increase during each pulse as well as with each subsequent pulse.

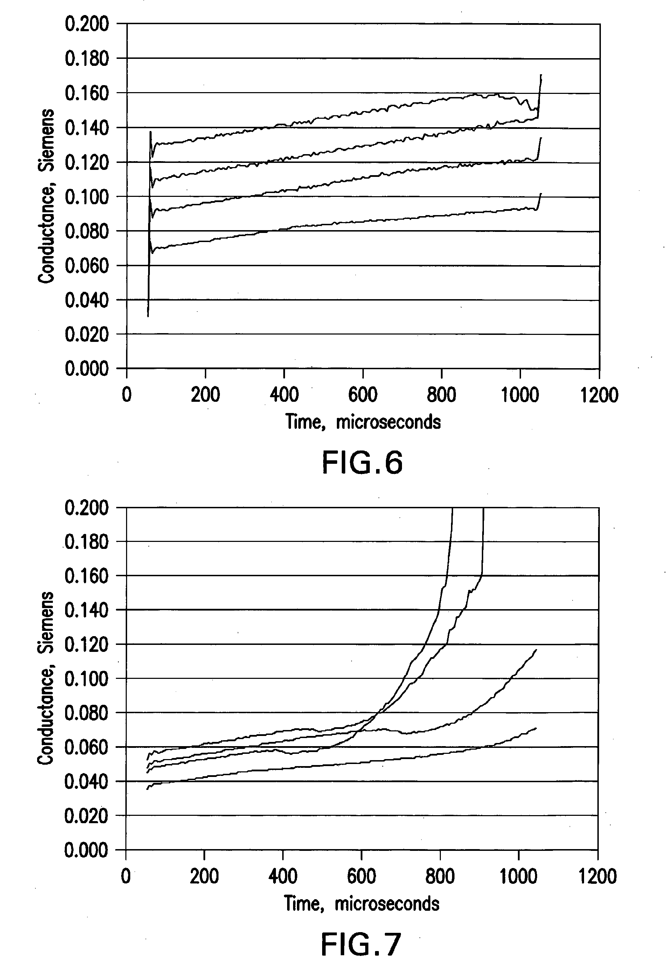

An electrical field of 1.5 kV/cm was applied in four consecutive pulses 1 ms duration 1 second apart to a conductive medium (FIG. 6). There is a noticeable conductivity increase during each pulse as well as with each subsequent pulse.

The conductivity values at the beginning of pulses 2-4 are approximately the same as the values at the end of the pulses before them. This illustrates slow rates of buffer cooling during the intervals between the pulses.

An electrical field of 2 kV/cm was applied in four consecutive pulses 1 ms duration 1 second apart to a conductive medium (FIG. 7). At this field strength the sample conductivity rapidly increased during each pulse, exceeding 200 percent of its initial value. Arcing occurred during the pulses 3 and 4.

### EXAMPLE 2

### PLATELET PREPARATION AND PROCESSING

**Platelet Isolation:** Platelets are isolated from PRP by first adjusting pH to 6.5 using 0.3 M citric acid -- 40 mL PRP + 400 µl Citric Acid and then centrifuging at 500 g for 15 min.

**Platelet Washing:** 2 wash cycles in Wash Buffer: 36 mM citric acid, 90 mM NaCl, 10 mM EDTA and 5 mM glucose, pH 6.5. Either PGE (at 1 µM final) or PGI (at 0.05 µg/mL final) is added prior to each wash.

**Platelet Preparation for EP:** After washing platelets are resuspended in 1 volume of MaxCyte EP Buffer (+2 mM EGTA) + 1 vol of 300 mM taurine (+2 mM EGTA). Platelet count is adjusted at 600K/µL.

**Platelet Resealing** EP and No-EP platelet suspensions are transferred into a water bath at 37°C for 30 min.

**Platelet Washing to remove the loaded compound:** 1 mL of Tyrode Buffer (135 mM NaCl, 4 mM KCl, 6 mM NaHCO$_3$, 0.5 mM NaH$_2$PO$_4$, 1 mM glucose, 10 mM HEPES, pH 6.5) is added to each sample tube containing the 150 µL of platelet suspension followed by centrifugation at 500 g for 10 min. 2 wash cycles with 1 mL of Tyrode Buffer (free of Ca++ and Mg++) at pH 6.5 are performed.

**Post-EP Analysis** After washing each aliquote of platelets is suspended in 300 µL PBS and analyzed by FACS.

### NUCLEIC ACID LOADING INTO PLATELETS

Platelets. Platelets were obtained from Lifeblood Biological Services (Memphis, TN). Platelets were isolated, washed and prepared for electroporation as described above.

Loading Platelets. Platelets were loaded with AllStars Negative Control siRNA (Qiagen, Inc.). The stock solution of siRNA was added to platelet suspension to final concentration of 1 μM, the sample was divided into "Coincubation" and "Electroporation" samples, and the latter was processed by application of electrical pulses on MaxCyte System. The power supply capacitance was set to 20 μF, field strength was 5 kV/cm.

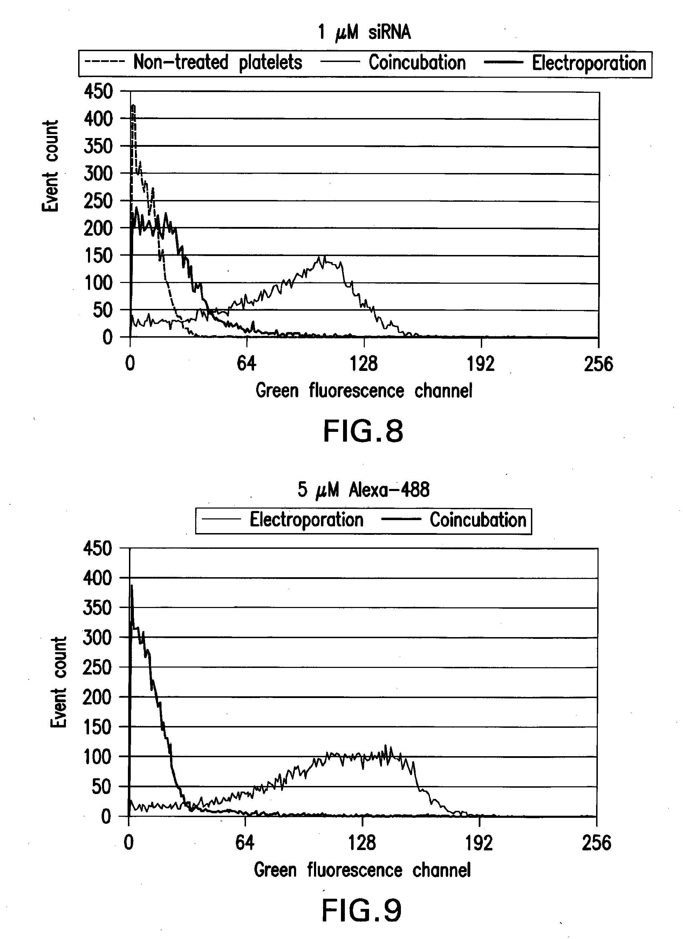

After electroporation the platelet suspensions were re-sealed and washed as described above. Results are illustrated in FIG. 8.

## EXAMPLE 3

### SMALL MOLECULE LOADING INTO PLATELETS

Platelets were prepared as previously described and loaded with Alexa-488 (Invitrogen, Inc.) by electroporation as described above. Results are illustrated in FIG. 9.

All of the compositions and/or methods disclosed and claimed herein can be made and executed without undue experimentation in light of the present disclosure. While the methods of this invention have been described in terms of particular embodiments, it will be apparent to those of skill in the art that variations may be applied to the compositions and/or methods in the steps or in the sequence of steps of the method described herein without departing from the scope of the invention, which is as defined by the appended claims. More specifically, it will be apparent that certain agents that are both chemically and physiologically related may be substituted for the agents described herein while the same or similar results would be achieved. All such similar substitutes and modifications apparent to those skilled in the art are deemed to be within the scope of the invention as defined by the appended claims.

## REFERENCES

The following references provide exemplary procedural or other details supplementary to those set forth herein:

U.S. Pat. No. 5,676,646
U.S. Pat. No. 7,186,559
Authi et al., FEBS Lett 254(1-2): 52-8, 1989.
Crawford and Chronos, Semin Interv Cardiol 1(1): 91-102, 1996
Hughes and Crawford, Biochim Biophys Acta 9.81(2): 277-87, 1989
Pfliegler et al., Thromb Haemost 72(4): 604-10, 1994
Rols and Teissie, Biophys J 75(3): 1415-23, 1998
Valeri et al., Transfusion, 45:1890-98, 2005.
Wolf et al., Biophys J 66(2 Pt 1): 524-31, 1994
WO1998/034478
US 2008 076144

**Claims**

1. A method for optimizing electroporation, the method comprising the steps of:

   determining a first time constant $t_1$ representative of electrical conductivity increase of an electroporation medium during an electric field pulse;
   determining a second time constant $t_2$ representative of a capacitor discharge in the electroporation medium;
   determining electroporation parameters such that $t_1$ is not less than $t_2$ and the pulse duration is less than either $t_1$ or $t_2$; and
   applying one or more electrical field pulses under the electroporation parameters to a sample to be electroporated in the electroporation medium.

2. The method of claim 1, wherein the electrical pulse is at least 1 μsec or the electrical pulse is of a magnitude of 0.001 to 10,000 V.

3. A method for optimizing electroporation, the method comprising the steps of:

   determining a rate of conductivity increase of an electroporation medium during an electrical field pulse;
   determining a rate of voltage decay across the electroporation medium during the pulse;
   determining electroporation parameters such that during a decaying electrical pulse the rate of conductivity increase of the electroporation medium is lower than the rate of voltage decay across the electroporation medium; and

applying one or more electrical pulses under the electroporation parameters to a sample to be electroporated in the electroporation medium.

**4.** The method of claim 1 or 3, wherein the sample comprises a living cell, a cell particle, or a lipid vesicle, in particular wherein the cell is a bacterium, a yeast cell, a blood cell or a platelet.

**5.** The method of claim 4, the method further comprising the step of loading the cell with a chemical or biological agent, in particular wherein the biological agent is a nucleic acid or small molecule.

**6.** The method of claim 1 or 3, wherein the electroporation parameters comprise buffer conductivity, power supply capacitance, electroporation chamber geometry, and electric field strength.

**7.** The method of claim 6, wherein the method is the method of claim 1 and wherein

(i) the buffer conductivity is 0 to 3 Ohm/m,
(ii) the power supply capacitance is between 1 and $10^6$ $\mu$F,
(iii) the electroporation chamber geometry includes a length of 0.1 to 100 cm, a width of 0.1 to 10 cm, and a gap of between 0.001 and 10 cm, or
(iv) the electric field strength is between 0.01 and 15 kV/cm.

**8.** The method of claim 3, wherein the rate of voltage decay is 10 $\mu$s to 10 s.

**9.** The method of claim 4 wherein the electroporated cells, cell particles, or lipid vesicles in the sample have a loading efficiency of at least 50, 60, 70, 80, or 90% after electrop oration.

**Patentansprüche**

**1.** Ein Verfahren zur Optimierung einer Elektroporation, wobei das Verfahren aus folgenden Schritten besteht:

Bestimmen einer ersten Zeitkonstante $t_1$, die für den Anstieg elektrischer Leitfähigkeit eines Elektroporationsmediums während eines Spannungsfeldimpulses repräsentativ ist;
Bestimmen einer zweiten Zeitkonstante $t_2$, die für eine Kondensatorentladung im Elektroporationsmedium repräsentativ ist;
Bestimmen von Elektroporationsparametern, so dass $t_1$ nicht weniger als $t_2$ beträgt und die die Impulsdauer weniger als entweder $t_1$ oder $t_2$ beträgt; und
Anwenden von einem oder mehreren Spannungsfeldimpulsen unter den Elektroporationsparametern auf eine Probe, die im Elektroporationsmedium elektroporiert werden soll.

**2.** Das Verfahren entsprechend Anspruch 1, wobei der elektrische Impuls zumindest 1 $\mu$Sek. beträgt oder der elektrische Impuls eine Stärke von 0,001 bis 10.000 V aufweist.

**3.** Ein Verfahren zur Optimierung einer Elektroporation, wobei das Verfahren aus folgenden Schritten besteht:

Bestimmen des Tempos eines Leitfähigkeitsanstiegs eines Elektroporationsmediums während eines Spannungsfeldimpulses;
Bestimmen des Tempos eines Spannungsabfalls über das Elektroporationsmedium während des Impulses;
Bestimmen von Elektroporationsparametern, so dass während eines abfallenden elektrischen Impulses das Tempo des Leitfähigkeitsanstiegs des Elektroporationsmediums niedriger als das Tempo des Spannungsabfalls über das Elektroporationsmedium ist; und
Anwenden von einem oder mehreren elektrischen Impulsen unter den Elektroporationsparametern auf eine Probe, die im Elektroporationsmedium elektroporiert werden soll.

**4.** Das Verfahren entsprechend Anspruch 1 oder 3, wobei die Probe eine lebende Zelle, ein Zellpartikel oder ein Lipidvesikel ist, insbesondere wobei die Zelle eine Bakterie, eine Hefezelle, eine Blutzelle oder ein Blutplättchen ist.

**5.** Das Verfahren entsprechend Anspruch 4, wobei zu dieser Methode weiterhin der Schritt des Ladens der Zelle mit einer Chemikalie oder einem biologischen Wirkstoff gehört, insbesondere wobei der biologische Wirkstoff eine Nukleinsäure oder ein kleines Molekül ist.

**6.** Das Verfahren entsprechend Anspruch 1 oder 3, wobei die Elektroporationsparameter Pufferleitfähigkeit, Stromzufuhrkapazität, Geometrie der Elektroporationskammer und Spannungsfeldstärke umfassen.

**7.** Das Verfahren entsprechend Anspruch 6, wobei es sich beim Verfahren um das Verfahren entsprechend Anspruch 1 handelt und wobei

(i) die Pufferleitfähigkeit 0 bis 3 Ohm/m beträgt,
(ii) die stromzufuhrstärke zwischen 1 und $10^6$ $\mu$F beträgt,
(iii) zur Geometrie der Elektroporationskammer eine Länge von 0,1 bis 100 cm, eine Breite von 0,1 bis 10 cm und eine Lücke von 0,001 und 10 cm gehört, oder
(iv) die Spannungsfeldstärke zwischen 0,01 und 15 kV/cm liegt.

**8.** Das Verfahren entsprechend Anspruch 3, wobei das Tempo des Spannungsabfalls 10 $\mu$s bis 10 s beträgt.

**9.** Das Verfahren entsprechend Anspruch 4, wobei die elektroporierten Zellen, Zellpartikel oder Lipidvesikel in der Probe eine Ladeleistung von mindestens 50, 60, 70, 80 oder 90% nach Elektroporation haben.


**Revendications**

**1.** Un procédé d'optimisation d'électroporation, le procédé comprenant les opérations suivantes :

la détermination d'une première constante de temps $t_1$ représentative d'une augmentation de conductivité électrique d'un milieu d'électroporation au cours d'une impulsion de champ électrique,
la détermination d'une deuxième constante de temps $t_2$ représentative d'une décharge de condensateur dans le milieu d'électroporation,
la détermination de paramètres d'électroporation de sorte que $t_1$ ne soit pas inférieur à $t_2$ et que la durée d'impulsion soit inférieure à soit $t_1$ ou $t_2$, et
l'application d'une ou de plusieurs impulsions de champ électrique selon les paramètres d'électroporation à un échantillon destiné à être électroporé dans le milieu d'électroporation.

**2.** Le procédé selon la Revendication 1, où l'impulsion électrique est d'au moins 1 $\mu$sec ou l'impulsion électrique est d'une magnitude de 0,001 à 10 000 V.

**3.** Un procédé d'optimisation d'électroporation, le procédé comprenant les opérations suivantes :

la détermination d'un taux d'augmentation de la conductivité d'un milieu d'électroporation au cours d'une impulsion de champ électrique,
la détermination d'un taux de décroissance de tension sur le milieu d'électroporation au cours de l'impulsion,
la détermination de paramètres d'électroporation de sorte qu'au cours d'une impulsion électrique décroissante, le taux d'augmentation de la conductivité du milieu d'électroporation soit inférieur au taux de décroissance de tension sur le milieu d'électroporation, et
l'application d'une ou de plusieurs impulsions électriques selon les paramètres d'électroporation à un échantillon destiné à être électroporé dans le milieu d'électroporation.

**4.** Le procédé selon la Revendication 1 ou 3, où l'échantillon comprend une cellule vivante, une particule cellulaire ou une vésicule lipidique, plus particulièrement où la cellule est une bactérie, une cellule de levure, une cellule sanguine ou une plaquette.

**5.** Le procédé selon la Revendication 4, le procédé comprenant en outre l'opération de chargement de la cellule avec un agent biologique ou chimique, plus particulièrement où l'agent biologique est un acide nucléique ou une petite molécule.

**6.** Le procédé selon la Revendication 1 ou 3, où les paramètres d'électroporation comprennent une conductivité de tampon, une capacité d'alimentation électrique, une géométrie de chambre d'électroporation et une intensité de champ électrique.

7. Le procédé selon la Revendication 6, où le procédé est le procédé selon la Revendication 1 et où

(i) la conductivité de tampon est de 0 à 3 Ohm/m,
(ii) la capacité d'alimentation électrique se situe entre 1 et $10^6$ $\mu$F,
(iii) la géométrie de la chambre d'électroporation comprend une longueur de 0,1 à 100 cm, une largeur de 0,1 à 10 cm et un espace situé entre 0,001 et 10 cm, ou
(iv) l'intensité de champ électrique se situe entre 0,01 et 15 kV/cm.

8. Le procédé selon la Revendication 3, où le taux de décroissance de tension est de 10 $\mu$s à 10 s.

9. Le procédé selon la Revendication 4 où les cellules électroporées, les particules cellulaires ou les vésicules lipidiques dans l'échantillon possèdent une efficacité de chargement d'au moins 50, 60, 70, 80 ou 90% après électroporation.

FIG.1

Current, A          4 kV cm⁻¹

FIG.2

Current, A          8 kV cm⁻¹

FIG.3

FIG.4

FIG.5

FIG.6

FIG.7

## 1 μM siRNA

---- Non-treated platelets ——— Coincubation ——— Electroporation

**FIG.8**

Green fluorescence channel

## 5 μM Alexa-488

——— Electroporation ——— Coincubation

**FIG.9**

Green fluorescence channel

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5676646 A
- US 7186559 B
- US 2008076144 A
- US 5720921 A
- US 5612207 A
- US 6623964 A
- US 20010001064 A
- US 4906576 A
- US 6897069 B
- WO 04083379 A
- WO 1998034478 A

**Non-patent literature cited in the description**

- **AUTHI et al.** *FEBS Lett,* 1989, vol. 254 (1-2), 52-8
- **CRAWFORD ; CHRONOS.** *Semin Interv Cardiol,* 1996, vol. 1 (1), 91-102
- **HUGHES ; CRAWFORD.** *Biochim Biophys Acta,* 1989, vol. 9.81 (2), 277-87
- **PFLIEGLER et al.** *Thromb Haemost,* 1994, vol. 72 (4), 604-10
- **ROLS ; TEISSIE.** *Biophys J,* 1998, vol. 75 (3), 1415-23
- **VALERI et al.** *Transfusion,* 2005, vol. 45, 1890-98
- **WOLF et al.** *Biophys J,* 1994, vol. 66, 524-31